# EUROPEAN PATENT APPLICATION

(11) **EP 4 834 251 A1**
(43) Date of publication of application: **04.11.2026**
(21) Application number: 25173855.5
(22) Date of filing: 01.05.2025
(51) Int. Cl.: A61B 17/3207, A61B 17/22

(54) **ROTATIONAL VIBRATING DEBULKING**

(71) Applicant: Koninklijke Philips N.V., 5656 AE Eindhoven (NL)
(72) Inventor: MILAITIS, Maxwell, Eindhoven (NL); MIEZWA, Megan Ann, Eindhoven (NL); MACCONNELL, Michael Bryan, 5656 AG Eindhoven (NL)
(74) Representative: Philips Intellectual Property & Standards

(57) **Abstract**

A catheter system includes a catheter having a distal end, a proximal end, and a longitudinal axis. The catheter system includes an inner shaft having a vibrational element disposed thereon; an outer shaft disposed around at least a proximal portion of the inner shaft; a middle shaft disposed between the inner shaft and the outer shaft; a plurality of blades, each having a proximal blade end and a distal blade end, the plurality of blades being disposed on the middle shaft and configured to radially expand and compress with respect to the longitudinal axis. The inner shaft and the middle shaft are configured to rotate about the longitudinal axis. The outer shaft is configured to translate with respect to the longitudinal axis in a longitudinal direction.

## Description

### BACKGROUND

Venous thromboembolism (VTE), which includes deep venous thrombosis (DVT), is a major contributor to global disease burden. VTE ranks as the third most common cardiovascular condition, following coronary artery disease and stroke. Lower extremity DVT (LEDVT) can obstruct the venous lumen, leading to venous congestion, swelling, and damage to the lower extremity venous valves, potentially resulting in post-thrombotic syndrome (PTS) if not quickly and adequately treated.

Standard treatment of venous obstructions includes the use of balloons, stents, lytics, and mechanical thrombectomy. Unfortunately, if DVT is not addressed early and progresses to PTS, stenting may be one of the few remaining treatment options available to physicians. However, implanted stents, especially in cases with concurrent inflow disease, are at risk of re-occlusion. Re-occluded stents have limited re-interventional options aside from surgical removal, which can significantly impair the patient's quality of life. Mechanical thrombectomy involves collection and extraction of the clot, either by mechanical entanglement and transport, or through use of vacuum aspiration. This method is only effective on acute and sub-acute thrombus. Presently, there are no effective endovascular methods to remove vascular lesions that have progressed in chronicity to PTS. In late PTS, the thrombus transforms into collagen which is tougher and more fibrous than thrombus and resistant to lytics. The presence of collagen causes vessel occlusion impeding blood flow and burdening the cardiovascular system. Moreover, this intraluminal collagen is nearly indistinguishable from the vessel wall, complicating its removal.

Accordingly, effective methods for endovascular removal of occlusions are needed.

### SUMMARY

According to an aspect of the present disclosure, a catheter system includes a catheter, a plurality of blades, and, optionally, a guide wire lumen. The catheter has a distal end and a proximal end and comprises a rotatable inner shaft having a vibrational element disposed thereon, a fixed outer shaft, and a rotatable middle shaft disposed between the rotatable inner shaft and the fixed outer shaft. The plurality of blades are fixed to the rotatable middle shaft and configured to expand and retract along the rotatable middle shaft. The guide wire lumen, if present, extends through the catheter.

According to an aspect of the present disclosure, a catheter system includes a catheter, a plurality of blades, and a vibrational element. The catheter has a distal end and a proximal end and includes a rotatable inner shaft and a fixed outer shaft. The plurality of blades are fixed to the fixed outer shaft and are configured to expand and retract along the fixed outer shaft. The vibrational element is disposed on the rotatable inner shaft.

According to another aspect of the present disclosure, a catheter system includes a catheter, a plurality of blades, a vibrational element, a handle, and a motor. The catheter has a distal end and a proximal end and includes a rotatable inner shaft and a fixed outer shaft. The plurality of blades are fixed to the fixed outer shaft and configured to expand and retract along the fixed outer shaft. The vibrational element is disposed on the rotatable inner shaft. The motor is disposed in the handle and configured to transfer rotational movement down the catheter via the rotatable inner shaft.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a system for rotational vibrating debulking, in accordance with a representative embodiment.
FIG. 2 illustrates a rotatable inner shaft for rotational vibrating debulking, in accordance with a representative embodiment.
FIG. 3 illustrates a system overview for rotational vibrating debulking, in accordance with a representative embodiment.
FIG. 4 illustrates rotational and vibrational motions for rotational vibrating debulking, in accordance with a representative embodiment.
FIG. 5A illustrates a debulking device in rotational vibrating debulking, in accordance with a representative embodiment.
FIG. 5B illustrates another debulking device in rotational vibrating debulking, in accordance with a representative embodiment.
FIG. 5C illustrates another debulking device in rotational vibrating debulking, in accordance with a representative embodiment.
FIG. 6A and FIG. 6B illustrate a system configuration with a handle with a motor, a power source, button and guide wire lumen in rotational vibrating debulking, in accordance with a representative embodiment.
FIG. 7 illustrates a drive shaft connected to blades for rotational vibrating debulking, in accordance with a representative embodiment.
FIG. 8 illustrates a blade control sheath with a circumferential groove for rotational vibrating debulking, in accordance with a representative embodiment.
FIG. 9 illustrates drive shaft rotating blades and a blade control sheath controlling size of the plurality of blades for rotational vibrating debulking, in accordance with a representative embodiment.
FIG. 10A, FIG. 10B and FIG. 10C illustrate side view of a tip, section view of a tip, and magnified view of rotational guide of blades for rotational vibrating debulking, in accordance with a representative embodiment.
FIG. 11 illustrates a second motor using a slot system to translate rotational movement into back-and-forth movement in rotational vibrating debulking, in accordance with a representative embodiment.
FIG. 12 illustrates shows another view of a second motor using a slot system to translate rotational movement into back-and-forth movement in rotational vibrating debulking, in accordance with a representative embodiment.
FIG. 13 illustrates centering wires in rotational vibrating debulking, in accordance with a representative embodiment.
FIG. 14 illustrates balloon centering in rotational vibrating debulking, in accordance with a representative embodiment.

### DETAILED DESCRIPTION

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a,' 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, a rotational vibrating debulking device may be provided as a catheter system with multiple blades which vibrate while rotating. The rotational vibrating debulking device will rotate via a motor in the proximal end of the catheter system. The motor translates rotational movement through the catheter system via a drive shaft to provide expansion and compression to a plurality of blades. The vibrations can induce increased separation of disease from the vessel wall. The vibrations may be produced by the rotating drive shaft by adding an eccentric rotating mass.

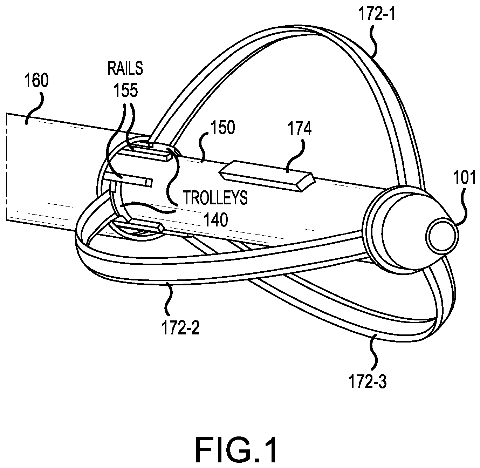

FIG. 1 illustrates a catheter system for rotational vibrating debulking, in accordance with a representative embodiment.

The catheter system in FIG. 1 and other FIGs. herein can be used endovascularly, and is a system for rotational vibrating debulking and includes components that may be provided together or that may be provided separately to be put together. The catheter system in FIG. 1 includes a rotatable inner shaft 150, a fixed outer shaft 160, a plurality of blades 172, and a vibrational element. To be sure, in other embodiments herein, another shaft is innermost, so beneath the rotatable inner shaft 150, such that the rotatable inner shaft 150 shown in FIG. 1 is a rotatable middle shaft and such that the innermost additional shaft has the vibrational element 174 thereon. The rotatable inner shaft 150 may be a drive shaft and has rails 155 integrally molded or otherwise fixedly attached thereto. Trolleys 140 are provided to be driven between sets of two of the rails 155. The trolleys 140 and/or the fixed outer shaft 160 may be actuated to slide along the rotatable inner shaft 150 and may be configured to push the plurality of blades 172 to project from the catheter once the catheter system is at the treatment zone. That is, the fixed outer shaft 160 and other actuator elements may be configured to translate motion along a longitudinal axis of the catheter system to provide expansion and compression to the plurality of blades 172. A set of actuator elements may include a motor in a handle, the fixed outer shaft 160, the trolleys 140, and other elements such as a detachable attachment between the fixed outer shaft 160 and one or more of the trolleys to enable withdrawal of the plurality of blades 172. An example of a detachable attachment is a detachable hook. The plurality of blades 172 includes a first blade 172-1, a second blade 172-2, and a third blade 172-3. Each of the plurality of blades 172 in FIG. 1 and corresponding pluralities of blades in other embodiments may be made of Nitinol or another shape-memory alloy. Nitinol is composed of nickel and titanium and is known for the ability to recover its original shape.

The catheter system in FIG. 1 also has a distal end and a proximal end. The catheter tip at the distal end of the catheter system in FIG. 1 includes a guide wire lumen 101. When the catheter system in FIG. 1 is navigated to a treatment system, the plurality of blades 172 are driven to project radially from the rotatable inner shaft 150 and relative to the fixed outer shaft 160, and then to rotate while projecting radially as the rotatable inner shaft 150 is rotated. That is, the actuator may be configured to push the plurality of blades 172 such that the plurality of blades 172 bow out from the rotatable inner shaft 150, and the plurality of blades 172 are rotated once projecting radially so as to perform debulking while the catheter system vibrates due to the presence and placement of the vibrational element 174. The rotation and vibration of the plurality of blades 172 as cutting elements is performed to remove a luminal obstruction within a diseased vessel. One or both leading edges of each of the plurality of blades 172 may be sharpened to cut material of the luminal obstruction when rotated clockwise and/or counterclockwise with the rotatable inner shaft 150. Each of the plurality of blades 172 vibrates during the rotation to improve cutting efficiency. The catheter system in FIG. 1 is designed to reestablish flow in an occluded vessel, such as a vein with post thrombotic scarring. The plurality of blades 172 will rotate and vibrate to cut the vascular lesion from the vessel wall to reopen the lumen.

An actuator is not labelled in FIG. 1, but is configured to drive the plurality of blades 172 to a treatment zone and then the motor, also not labelled in FIG. 1, rotates the plurality of blades 172 once the catheter system in FIG. 1 is at the treatment zone. The actuator may comprise the trolleys 140 and/or the fixed outer shaft 160. Each of the plurality of blades 172 is compressible and expandable for tracking over the rotatable inner shaft via the rails 155 and deployment through the vessel. The plurality of blades 172 are rotated by a motor in a handle which transfers rotational movement down the catheter system. The motor in the handle is configured to transfer rotational movement down the catheter system in FIG. 1 via the rotatable inner shaft 150. The plurality of blades 172 are configured to rotate and be disposed substantially flat along the rotatable inner shaft 150 while the catheter system is navigated to the treatment zone and to then be driven by the actuator (not labelled) to project from the catheter system radially once the catheter system is at the treatment zone. The catheter system in FIG. 1 may also include a guide wire through the guide wire lumen 101 configured to project from the distal end of the catheter system while the catheter system is navigated to the treatment zone.

The plurality of blades 172 are cutting elements and are configured to project radially from the rotatable inner shaft 150 of the catheter system in FIG. 1 by bowing out when compressed by the actuator (not labelled) pushing the trolleys 140 along the rails 155. In other words, the plurality of blades 172 are activated to project radially by being compressed by the trolleys 140 on one end so as to bow out from the rotatable inner shaft 150 once at a treatment zone, and then rotated with the rotatable inner shaft 150 to perform the debulking. The amount of bowing may be increased from the start of debulking so as to progressively debulk matter from the middle of a vessel to the inner periphery of the vessel, so progressively radially outward. To activate the plurality of blades 172, the plurality of blades 172 are compressed which causes the plurality of blades 172 to bow out as seen in FIG. 1. The radiuses of the plurality of blades 172 are increased by the compression. Radius of the plurality of blades 172 may be controlled by movement of the fixed outer shaft 160 along the catheter system in FIG. 1. The radiuses of each blade of the plurality of blades 172 are substantially constant when flat along the rotatable inner shaft 150, but will substantially increase except at the anchor points at the trolleys 140 and the opposing anchor points at the distal end of the catheter system in FIG. 1. The radiuses will also vary along the blades once subject to the compression. This will allow for use in multiple vessel locations and increase effectiveness of cutting. The largest radius of bowing relative to the rotatable inner shaft 150 may be at the center of each blade of the plurality of blades, and the radius at and from the center may be varied to increase during a bulking process. 11. An amount of projection of any one of the plurality of blades 172 from the catheter system in FIG. 1 can be varied to change a radius of the plurality of blades 172. The increase in radius relative to the rotatable inner shaft 150 may correspond to a reduction in minimal distance between the two ends of any one of the plurality of blades 172 as one of the trolleys 140 pushes one end of the one of the plurality of blades 172 along the rails. 155.

The vibrational element 174 is a weight disposed on top of the rotatable inner shaft 150 close to the distal tip of the rotatable inner shaft 150 which ends at the guide wire lumen 101. The vibrational element 174 is offset from a geometric center of the rotatable inner shaft 150. The vibrational element 174 may comprise an eccentric rotating mass configured to translate vibrations to the plurality of blades 172. For example, the vibrational element 174 may comprise an offset weight by which a center of mass of the catheter system in FIG. 1 is shifted to be offset from a center of the catheter system in FIG. 1. In some embodiments, the vibrational element 174 may be centered at one of the plurality of blades 172 to provide consistent vibrations across the plurality of blades 172. The vibrational element 174 in FIG. 1 is disposed along the distal end of the catheter system in FIG. 1 and near the plurality of blades 172. In other embodiments, the vibrational element 174 may be disposed on the proximal end of a catheter system modified relative to FIG. 1. In some embodiments, the vibrational element 174 may comprise a piezoelectric transducer, a shape memory alloy, or an electroactive polymer. The vibrational element 174 in FIG. 1 and corresponding elements of other embodiments may also be implemented using a higher density element in the rotatable inner shaft 150. For example, a relatively higher density element than the rest of the rotatable inner shaft may be set in the molding along some or all of 20° -180° along the circumference of the rotatable inner shaft 150.

In a modified embodiment based on FIG. 1, the vibrational element 174 is an eccentric rotating element closer to the proximal end of the rotatable inner shaft 150 rather than closer to the distal end. In the modified embodiment, the vibrational element 174 may be located near or in the handle and the handle is provided with vibrational dampening qualities. A motor in the handle may be configured to transfer rotational movement down the catheter system in FIG. 1 via the rotatable inner shaft, and the vibrations travel down the catheter body to the debulking tip at the rightmost extremity of the rotatable inner shaft 150. The weight may be provided outside of the area around the plurality of blades 172 in the modified embodiment.

As set forth above, a rotational vibrating debulking device shown as the catheter system in FIG. 1 is provided with a plurality of blades 172 which vibrate while rotating. The rotational vibrating debulking device may rotate via a motor (not shown) in the proximal end of the catheter system. The motor transmits rotational movement through the catheter system via the rotatable inner shaft 150. The plurality of blades 172 may be attached to the rotatable inner shaft 150 and rotate with the rotatable inner shaft 150 as the motor is activated. The vibrations can induce increased separation of disease from the vessel wall. The vibrations will be produced by the rotatable inner shaft 150 by adding the vibrational element 174 as an eccentric rotating mass. The weight may be near to the plurality of blades 172 to ensure proper translation of vibrations to the plurality of blades 172. By adding an offset weight to the rotatable inner shaft 150, the center of mass by the plurality of blades 172 will not be at center of rotation causing imperfect rotations. To have consistent vibrations across the plurality of blades 172, the weight of the vibrational element 174 may be placed centered at or near one of the plurality of blades 172.

In FIG. 1, a catheter system for rotational vibrating debulking will vibrate due to the offset of a weight as the vibrational element 174 at the tip of the rotatable inner shaft 150. The weight is fixed and located in between two of the plurality of blades 172. The weight will move the center of mass from center of rotation. This offset creates a vibrating effect.

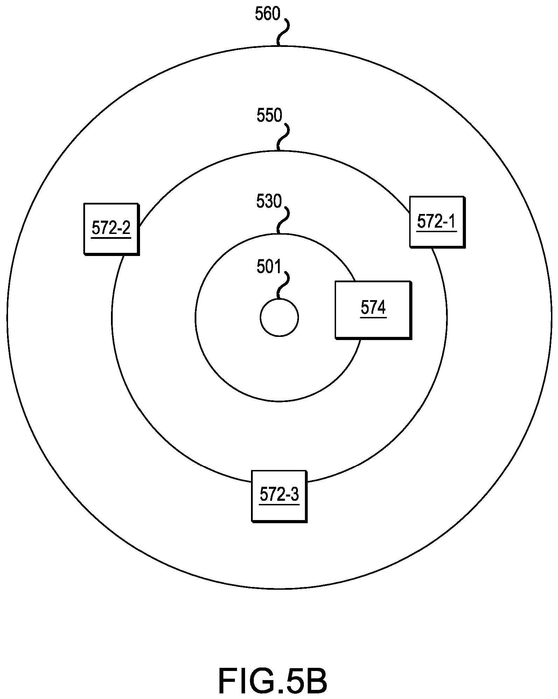

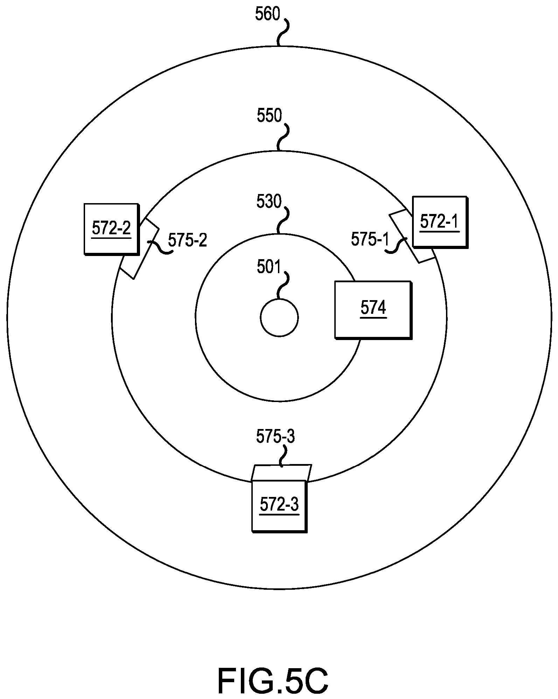

In FIG. 1, two shafts are provided including the rotatable inner shaft 150 and the fixed outer shaft 160. The rotating inner shaft 150 has both the plurality of blades 172 and the vibrating element 174 disposed thereon. In other embodiments, two rotating inner shafts may be provided, so a rotating inner shaft and a rotating middle shaft. The rotating inner shaft and rotating middle shaft in the other embodiments allow for separately vibrating the vibrating element 174 independent of rotating the plurality of blades 172. A method operating the catheter system in claim 1 may include separately initiating vibration by the vibrational element and rotation of the plurality of blades. The plurality of blades 172 may be provided on one of the two rotating inner shafts, and the vibrating element 174 may be provided on the other of the two rotating inner shafts. Examples with two rotating inner shafts used to divide the functionality of the plurality of blades and the vibrating element are shown in FIG. 5B and in FIG. 5C.

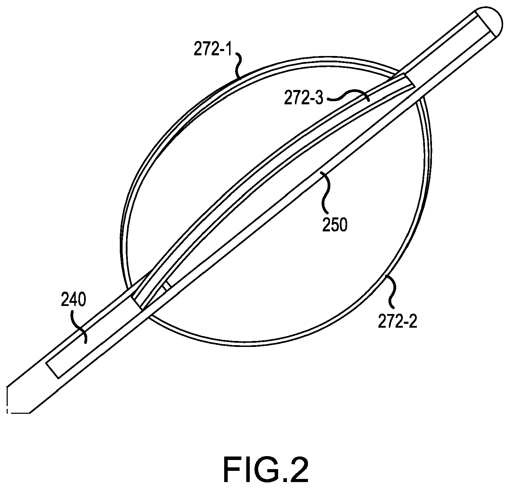

FIG. 2 illustrates a rotatable inner shaft for rotational vibrating debulking, in accordance with a representative embodiment.

The rotatable inner shaft 250 in FIG. 2 is provided with a plurality of blades 272 and a trolley 240. The plurality of blades include a first rotatable blade 272-1, a second rotatable blade 272-2, and a third rotatable blade 272-3. As shown in FIG. 2, the plurality of blades 272 are activated to project radially from the rotatable inner shaft 250. The plurality of blades 272 may be carried to a treatment site in a deactivated state while flat against the rotatable inner shaft 250, then activated at the treatment site, then rotated while activated. The amount of radial projection may be adjusted to increase from start to finish at a position at the treatment site, so as to reduce the possibility that the plurality of blades 272 becoming snagged. Although FIG. 2 does not show a vibrational element, a vibrational element on the rotatable inner shaft 250 causes vibrations when the rotatable inner shaft 250 is rotated, and the vibrations propagate to and through the plurality of blades 272 so as to enhance removal of the material being cut by the plurality of blades 272.

FIG. 2 also shows a trolley 240 between rails (unlabeled) to push the third rotatable blade 272-3 to project radially from the rotatable inner shaft 250. The trolley 240 may be detachably attached to a fixed outer shaft to retract the plurality of blades 272. For example, a hook may be used as a detachable attachment.

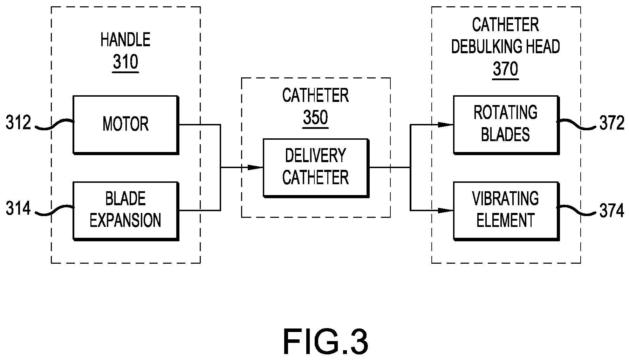

FIG. 3 illustrates a system overview for rotational vibrating debulking, in accordance with a representative embodiment.

The system overview in FIG. 3 includes a handle 310, a catheter 350, and a catheter debulking head 370. These three elements may be integrated as a single unit, as two units of the handle 310 as a first unit and the catheter 350 and catheter debulking head 370 as a second unit, or as three separate units. The handle 310 includes a motor 312 and a blade expansion 314 as an actuator. The catheter 350 comprises a delivery catheter. The motor 312 in the handle 310 is configured to transfer rotational movement down the catheter to the catheter debulking head 370 via a rotatable inner shaft. The catheter debulking head 370 includes rotating blades 372 and a vibrating element 374.

The catheter system in FIG. 3 is a rotational vibrating debulking device which is flexible and which can be used endovascularly. The catheter system is configured to debulk vascular lesions due to the expandable radial cutting elements of the rotating blades 372 combined with the vibrating element 374. The catheter system in FIG. 3 and other embodiments herein is configured to remove vascular lesions via rotating and vibrating the plurality of blades 372. Vascular lesions protrude between the rotating blades 372 while they are rotating, allowing for the rotating blades to engage the diseased tissue. The vibrations are produced by one or more offset weight(s) provided by the vibrating element 374 along the distal end of the catheter system near the rotating blades 372. The rotational vibrating debulking device enhances separation of disease from the vessel wall due to the vibrations. The vibrations will help move the layers of tissue while the rotating blades 372 are rotating. The vibrations also create back and forth movement, increasing the effectiveness of the de-bulking while limiting the force of the rotating blades 372. With the assisted cutting, the catheter system in FIG. 3 reduces the chance of snagging the disease instead of cutting it, resulting in over torquing a rotatable inner shaft used as the drive shaft. The over torquing can cause a drive shaft to twist and fail. Additionally, if the catheter system in FIG. 3 catches the disease, the catheter system may twist the vessel, possibly causing damage or vessel perforation. The assisted cutting reduces the possibility of causing these problems due to the enhanced cutting from the rotating blades 372 and the vibrations from the vibrating element 374.

Although not shown in FIG. 3 or other embodiments, material cut by the rotating blades 372 may be caught by a filter placed upstream or downstream from the treatment site. A filter may take the shape of a basket. Alternatively, a vacuum element may be used in some embodiments to capture material cut by the rotating blades 372. A filter, vacuum or other mechanism to capture cut material may be used in any of the embodiments described herein.

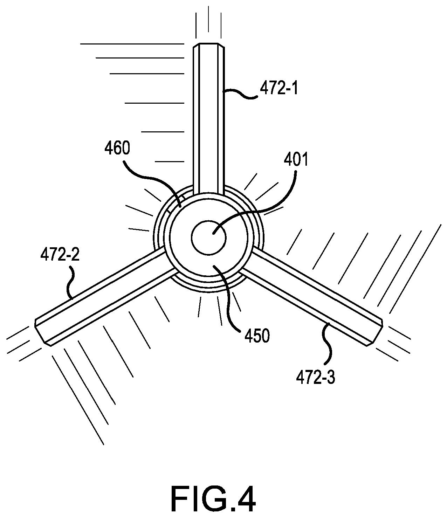

FIG. 4 illustrates rotational and vibrational motions for rotational vibrating debulking, in accordance with a representative embodiment.

A catheter system in FIG. 4 includes a rotatable inner shaft 450, a fixed outer shaft 460, and a plurality of blades 472 including a first rotatable blade 472-1, a second rotatable blade 472-2, and a third rotatable blade 472-3. Rotation in FIG. 4 is clockwise. The rotational motions are from the trailing edges of the plurality of blades 472 such as the lines designating trailing motions from the trailing edge of the first rotatable blade 472-1. The vibrational motions are those emanating radially from the center of the catheter system, such as those projecting from the fixed outer shaft 460. The fixed outer shaft 460 and other actuator elements may be configured to translate motion along a longitudinal axis of the catheter system to longitudinally to provide expansion and compression to the plurality of blades 472 which are then enabled to rotate. A set of actuator elements may include a motor in a handle, the fixed outer shaft 460, trolleys, and other elements such as a detachable attachment between the fixed outer shaft 460 and one or more of the trolleys to enable withdrawal of the plurality of blades 472. For example, a hook may be used as a detachable attachment.

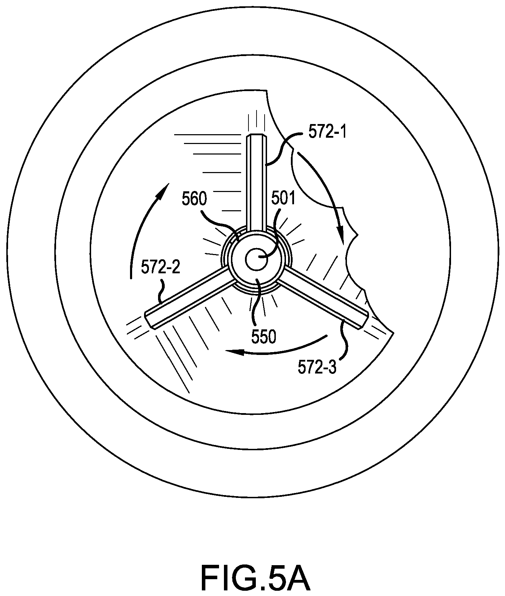

FIG. 5A illustrates a debulking device in rotational vibrating debulking, in accordance with a representative embodiment.

The catheter system in FIG. 5A includes a rotatable shaft 550, a fixed outer shaft 560, and a plurality of blades 572 including a first rotatable blade 572-1, a second rotatable blade 572-2, and a third rotatable blade 572-3.The rotational motion in FIG. 5A is also shown as clockwise, and material to be removed is shown between the first rotatable blade 571-1 and the third rotatable blade 572-3. The maximum projection radius of each of the plurality of blades 572 may be slowly increased as a way to incrementally remove material in a vessel. Radius of the plurality of blades 572 may be controlled by movement of the fixed outer shaft 560 along the catheter system in FIG. 5A. Additionally, the rotatable shaft 550 may be projected slowly closer to and further from the fixed outer shaft 560 to adjust the areas in the vessel of the center portions of each of the plurality of blades 572. The fixed outer shaft 560 and other actuator elements may be configured to translate motion along a longitudinal axis of the catheter system to push and longitudinally compress the plurality of blades 572 which are then enabled to rotate. A set of actuator elements may include a motor in a handle, the fixed outer shaft 560, trolleys, and other elements such as a detachable attachment between the fixed outer shaft 560 and one or more of the trolleys to enable withdrawal of the plurality of blades 572. For example, a hook may be used as a detachable attachment.

The rotation and vibration in the catheter systems in FIG. 5A and other embodiments herein combine to clear vascular lesions such as DVT. Late stage DVT organizes into a tough collagen-based disease state which renders most thrombectomy devices useless. In cases such as PTS where collagen based lesion is morphologically similar to the vessel wall, thrombectomy devices that use differences between the vessel and properties of the disease are ineffective in removing the disease. Similarly, atherectomy and thrombectomy devices which use forward facing cutting techniques are not designed to cut luminal scar tissue, cannot differentiate between cutting vascular lesions and the vessel wall, and tend to lack the ability to treat larger diameter vessels. Aspiration technologies in the thrombectomy market are also not sufficient for these lesions at this stage of chronicity. For example, aspiration thrombectomy devices do not work due to the tough nature of collagen and strong adhesion to the endothelial wall, wherein the aspiration is not strong enough to pull the collagen from the wall. The combined rotation and vibration of catheter systems described herein work to clear DVT including cases such as PTS. The catheter systems herein are suitable for endovascular procedures due to their flexibility of both the fixed outer shafts, rotatable inner shafts, and plurality of blades.

FIG. 5B illustrates another debulking device in rotational vibrating debulking, in accordance with a representative embodiment.

In FIG. 5B, a catheter system is shown in profile. The catheter system in FIG. 5B includes a rotatable inner shaft 530 having a vibrational element 574 disposed thereon, a fixed outer shaft 560, and a rotatable shaft 550 disposed between the rotatable inner shaft 530 and the fixed outer shaft 560. Relative to the embodiment of FIG. 5A, the rotatable inner shaft 530 is added to separately have the vibrational element 574, whereas the rotatable shaft 550 is comparable to the rotatable inner shaft in the embodiment of FIG. 5A. In FIG. 5B, the plurality of blades 572 are fixed to the rotatable shaft 550 and configured to expand and retract along the rotatable shaft 550 rather than along a rotatable inner shaft as in FIG. 5A. The rotatable shaft 550 in FIG. 5B may include rails (not shown) and a trolley (not shown) to enable movement of the plurality of blades 572. In other words, in embodiments such as FIG. 5A, a single rotatable inner shaft may have both the vibrational element and the plurality of blades, whereas in FIG. 5B the vibrational element 574 is provided on a rotatable inner shaft 530 which is innermost and the plurality of blades 572 are provided on a rotatable shaft 550. The rotatable inner shaft 530 and rotatable shaft 550 (rotating middle shaft) in FIG. 5B allow for separately vibrating the vibrating element 574 independent of rotating the plurality of blades 572. An actuator such as trolleys (not shown) and/or the fixed outer shaft 560 may be configured to push the plurality of blades 572 such that the plurality of blades 572 bow out from the rotatable shaft 550. That is, the fixed outer shaft 560 and other actuator elements may be configured to translate motion along a longitudinal axis of the catheter system to push and longitudinally compress the plurality of blades 572 which are then enabled to rotate. In FIG. 5B, a guide wire lumen 501 extends through the catheter system. Additionally, a handle disposed at the proximal end of the catheter system has an actuator configured to push the plurality of blades 572 such that the plurality of blades 572 bow out from the rotatable shaft 550.

In FIG. 5B, the catheter system may also have a first motor operatively connected to the rotatable inner shaft 530 and configured to rotate the rotatable inner shaft 530 to generate or otherwise transmit vibrations. A second motor may also be operatively connected to the rotatable shaft 550, wherein the rotatable shaft 550 is connected to the plurality of blades 572, and the second motor is configured to rotate the rotatable shaft 550 to thereby rotate the plurality of blades 572. In other embodiments based on FIG. 5B, the rotatable inner shaft 530 and the rotatable shaft 550 are operatively connected to a single motor, such that the single motor is configured both to rotate the rotatable inner shaft 530 to generate or otherwise transmit vibrations and to rotate the rotatable shaft 550 to thereby rotate the plurality of blades 572. The single motor may be configured to rotate both the rotatable inner shaft 530 at a first rotation speed to generate vibrations in the plurality of blades 572 and to rotate the rotatable shaft 550 at a second rotation speed to thereby rotate the plurality of blades 572. In the embodiments with a single motor, the single motor may be disposed in the handle.

FIG. 5C illustrates another debulking device in rotational vibrating debulking, in accordance with a representative embodiment.

In FIG. 5C, a catheter system similar to the catheter system is again shown in profile. The catheter system in FIG. 5C includes the rotatable inner shaft 530 having the vibrational element 574 disposed thereon, the fixed outer shaft 560, and the rotatable shaft 550 disposed between the rotatable inner shaft 530 and the fixed outer shaft 560. Relative to the embodiment of FIG. 5B, the a first recess 575-1 is shown in the rotatable shaft 550, a second recess 575-2 is shown in the rotatable shaft 550, and a third recess 575-3 is shown in the rotatable shaft 550. The plurality of blades 572 are retracted in and stored in the recesses while the catheter system is conveyed to a treatment site, and then drawn out of the recesses once at the treatment site. The rotatable shaft 550 in FIG. 5C may also include rails (not shown) and a trolley (not shown) to enable movement of the plurality of blades 572 to move in and out of the recesses. The vibrational element 574 is again on a different rotatable shaft than the plurality of blades 572. In FIG. 5C, the plurality of blades 572 are again fixed to the rotatable shaft 550 and configured to expand and retract along the rotatable shaft 550 rather than along a rotatable inner shaft as in FIG. 5A. In FIG. 5C the vibrational element 574 is provided on a rotatable inner shaft 530, which is innermost and the plurality of blades 572 are provided on a rotatable shaft 550. The rotatable shaft 550 may be considered a rotatable outer shaft relative to the rotatable inner shaft 530, but may be considered another (i.e., second) rotatable shaft relative to the fixed outer shaft 560. The rotatable inner shaft 530 and rotatable shaft 550 (rotating middle shaft) in FIG. 5C allow for vibrating the vibrating element 574 independent of rotating the plurality of blades 572.

An actuator such as trolleys (not shown) and/or the fixed outer shaft 560 may be configured to push the plurality of blades 572 such that the plurality of blades 572 bow out from the rotatable shaft 550. That is, the fixed outer shaft 560 and other actuator elements may be configured to translate motion along a longitudinal axis of the catheter system to push and longitudinally compress the plurality of blades 572 which are then enabled to rotate. In FIG. 5C, a guide wire lumen 501 again extends through the catheter system. Additionally, a handle disposed at the proximal end of the catheter system has an actuator configured to push the plurality of blades 572 such that the plurality of blades 572 bow out from the rotatable shaft 550.

In FIG. 5C, the catheter system may also have a first motor in a handle and operatively connected to the rotatable inner shaft 530 and configured to rotate the rotatable inner shaft 530 to generate or otherwise transmit vibrations. A second motor in a handle may also be operatively connected to the rotatable shaft 550, wherein the rotatable shaft 550 is connected to the plurality of blades 572, and the second motor is configured to rotate the rotatable shaft 550 to thereby rotate the plurality of blades 572. In other embodiments based on FIG. 5C, the rotatable inner shaft 530 and the rotatable shaft 550 are operatively connected to a single motor. In these other embodiments, the single motor is configured both to rotate the rotatable inner shaft 530 to generate or otherwise transmit vibrations and to rotate the rotatable shaft 550 to thereby rotate the plurality of blades 572. The single motor may be configured to rotate both the rotatable shaft 550 at a first rotation speed to generate vibrations in the plurality of blades 572 and to rotate the rotatable shaft 550 at a second rotation speed to thereby rotate the plurality of blades 572. In the embodiments with a single motor, the single motor may be disposed in the handle.

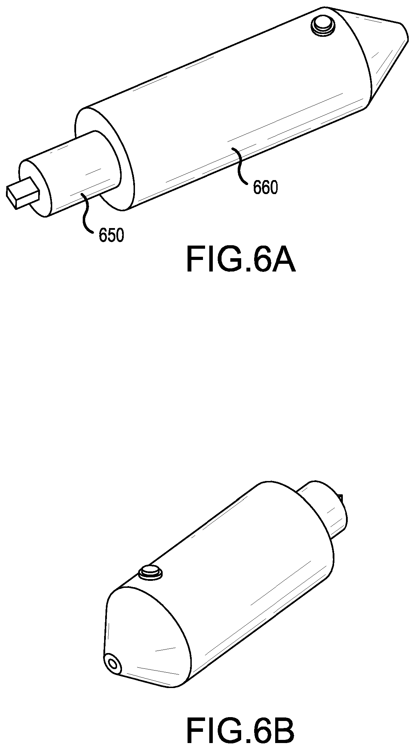

FIG. 6A and FIG. 6B illustrate a system configuration with a handle with a motor, a power source, button and guide wire lumen in rotational vibrating debulking, in accordance with a representative embodiment.

In FIG. 6A and FIG. 6B, a rotatable inner shaft 650 is shown projecting from a fixed outer shaft 660. The rotational vibrating debulking device in FIG. 6A and FIG. 6B has a motor on the handle to transmit rotational movement down the catheter. The motor speed may be fixed for the user and will start rotating at governed speed such as by the pressing of a button as seen on the top in both views. To power the motor, a power source may be connected. A power source in FIG. 6A and FIG. 6B is designated by the tubular piece on the most proximal side of the catheter. The motor will have a lumen for the guide wire lumen on the far right of the left view and on the far left of the right view. The motor may be concentric with the guide wire lumen. Although not labelled, the handle in FIG. 6A and FIG. 6B may also have a control such as a knob and/or button and/or sliding mechanism for adjusting diameter of a plurality of blades once at a treatment site so that the plurality of blades can be controlled to bow out. A power source for the motor may be a wire such that the handle has an interface such as a plug, or may be a battery including a rechargeable battery.

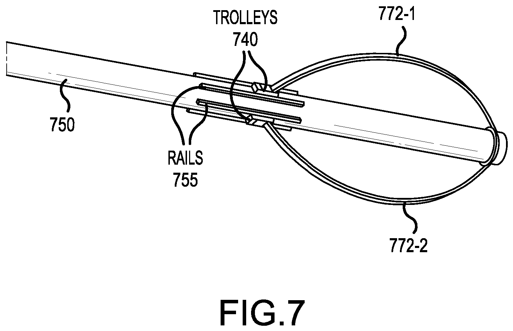

FIG. 7 illustrates a drive shaft connected to blades for rotational vibrating debulking, in accordance with a representative embodiment.

In FIG. 7, a drive shaft is shown as the rotatable inner shaft 750. Rails 755 are provided on the rotatable inner shaft 750. Trolleys 740 are provided between the rails 755 and connected to first ends of the first rotatable blade 772-1 and the second rotatable blade 772-2. Second ends of the first rotatable blade 772-1 and the second rotatable blade 772-2 are fixed to the distal-most end of the rotatable inner shaft 750.

To translate the rotation down the catheter in FIG. 7, a rotatable inner shaft 750 may be constructed as a drive shaft from a laser cut hypo tube that resists twisting motions, allowing for rotational movement to be translated down the rotatable inner shaft 750 to the plurality of blades 772 which are rotating blades. Additionally, a laser cut hypo tube may be configured to bend allowing it to curve around the turns of the vessel and maintain the strength to translate the rotational movement down the catheter. A guide wire will be able to pass through a guide wire lumen at the center of the rotatable inner shaft 750.

The rotatable inner shaft connects to one or more blades at the distal end of the catheter. Each blade of the plurality of blades may have a fixed distal tip as shown on the far right in FIG. 7. A ring around the distal tip of a rotatable outer shell (not shown) may connect to each of the plurality of blades 772. The proximal end of each of the plurality of blades 772 is free to move along the catheter, but rotational movement is guided by the rails 755 on the rotatable inner shaft 750.

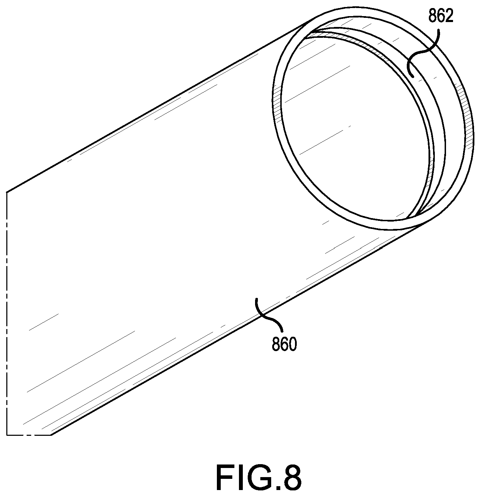

FIG. 8 illustrates a blade control sheath with a circumferential groove for rotational vibrating debulking, in accordance with a representative embodiment. In FIG. 8, a fixed outer shaft 860 includes a circumferential groove 862 on an inner surface. To collapse and expand the plurality of blades, the fixed outer shaft 860 may be constructed from a hypo tube to be placed over a rotatable inner shaft serving as a drive shaft. The fixed outer shaft 860 contains and protects the drive shaft while controlling the plurality of blades as a blade control sheath to allow the plurality of blades to rotate freely but control the movement along the rotatable inner shaft of a catheter system. The fixed outer shaft 860 may be configured to contact at least one of a plurality of blades at the distal end of a catheter. A protrusion on top of the proximal end of each of the plurality of blades will be guided by a circumferential groove around the interior of the fixed outer shaft 860 or a rotatable outer shaft or middle shaft as in FIG. 5B and FIG. 5C. The circumferential groove 862 in the fixed outer shaft 860 is configured to engage protrusions on top of the proximal end of a plurality of blades, wherein radius of the plurality of blades is controlled by movement of the fixed outer shaft 860 along the catheter system. The radius of the plurality of blades from the center of the rotatable inner shaft may be controlled by the movement of the fixed outer shaft 860 along the catheter. When the fixed outer shaft 860 moves forward the plurality of blades are pushed to bow more, increasing the radius. In transport to the disease state, the plurality of blades are pulled back causing each of the plurality of blades to collapse and form straight blades.

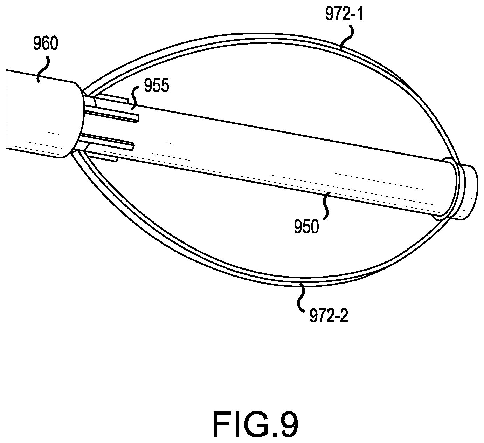

FIG. 9 illustrates drive shaft rotating blades and a blade control sheath controlling size of the plurality of blades for rotational vibrating debulking, in accordance with a representative embodiment.

In FIG. 9, a catheter system includes a rotatable inner shaft 950 projecting from a fixed outer shaft 960. Rails 955 are provided on the rotatable inner shaft 950 so that trolleys which are unlabeled can be moved along the rails 955 to move the plurality of blades 972. A first rotatable blade 972-1 and a second rotatable blade 972-2 are shown projecting from movable trolleys on a first end, and fixed to the distal-most end of the rotatable inner shaft 950 on a second end opposite to the first end. The trolleys and/or the fixed outer shaft 960 along with a motor in a handle may comprise an actuator slidable along the rotatable inner shaft 950 using the rails 955 and configured to push the plurality of blades 972 to project from the catheter system once the catheter system is at the treatment zone. The fixed outer shaft 960 is a sheath that controls size of the plurality of blades 972 by pushing them directly on the proximal side along the rails fixed to the rotatable inner shaft 950. The bowing of the plurality of blades 972 shows that they are at least partially pushed by the fixed outer shaft 960, but the exposed portions of the rails 955 shows that the plurality of blades 982 are not compressed as much as they could be.

In FIG. 9, two shafts are provided including the rotatable inner shaft 950 and the fixed outer shaft 960. The rotating inner shaft 950 has both the plurality of rails 955 and the vibrating element (not labelled) disposed thereon. The fixed outer shaft 960 and other actuator elements may be configured to translate motion along a longitudinal axis of the catheter system to push and longitudinally compress the plurality of blades 972 which are then enabled to rotate. In other embodiments, two rotating inner shafts may be provided, so a rotating inner shaft and a rotating middle shaft. The plurality of blades 972 may be provided on one of the two rotating inner shafts, and the vibrating element may be provided on the other of the two rotating inner shafts. In these other embodiments, the fixed outer shaft 960 and other actuator elements still translate motion longitudinally to push the plurality of blades 972. An example with two rotating inner shafts used to divide the functionality of the plurality of blades and the vibrating element was shown in FIG. 5B.

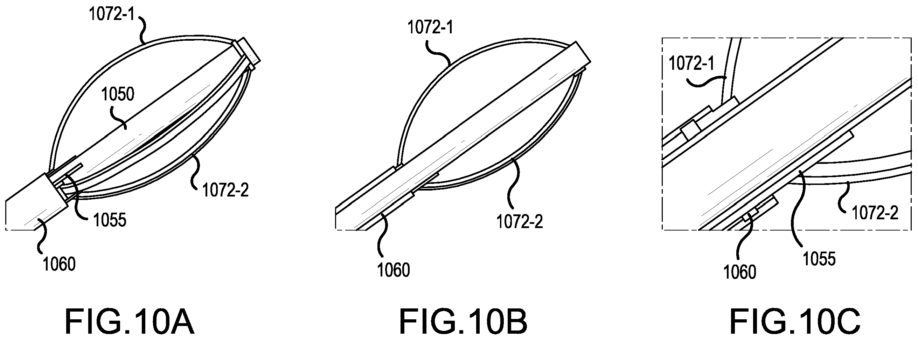

FIG. 10A, FIG. 10B and FIG. 10C illustrate side view of a tip (FIG. 10A), section view of a tip (FIG. 10B), and magnified view of rotational guide of blades (FIG. 10C) for rotational vibrating debulking, in accordance with a representative embodiment.

The three views in FIG. 10A, FIG. 10B and FIG. 10C show a catheter system that includes a first rotatable blade 1072-1 and a second rotatable blade 1072-2 attached on one end to and otherwise projecting from a rotatable inner shaft 1050. A fixed outer shaft 1060 is shown also to demonstrate that the rotatable inner shaft 1050 is projecting from the fixed outer shaft 1060. Rails 1055 are provided on the rotatable inner shaft 1050 so that trolleys which are unlabeled can be moved along the rails 1055 to move the plurality of blades 1072.

In FIG. 10A, FIG. 10B and FIG. 10C, a catheter system for rotational vibrating debulking will vibrate due to the offset of a weight at the tip of the catheter. The weight is fixed and located in between two of the blades. The weight will move the center of mass from center of rotation. This offset creates a vibrating effect. The weight will go along the catheter as seen in figures showing the vibrational element on the drive shaft such as the rotatable inner shaft 1050 in FIG. 10A, FIG. 10B and FIG. 10C.

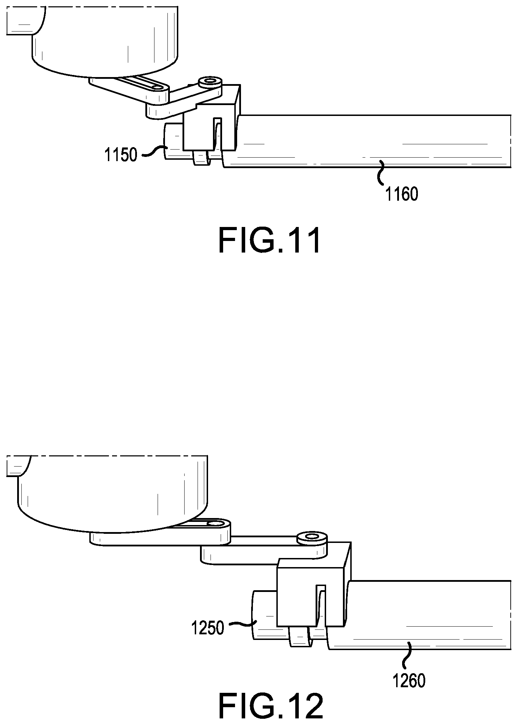

FIG. 11 illustrates a second motor using a slot system to translate rotational movement into back-and-forth movement in rotational vibrating debulking, in accordance with a representative embodiment.

FIG. 11 shows a rotatable inner shaft 1150 projecting from a fixed outer shaft 1160.

FIG. 12 illustrates shows another view of a second motor using a slot system to translate rotational movement into back-and-forth movement in rotational vibrating debulking, in accordance with a representative embodiment.

FIG. 12 shows a rotatable inner shaft 1250 projecting from a fixed outer shaft 1260.

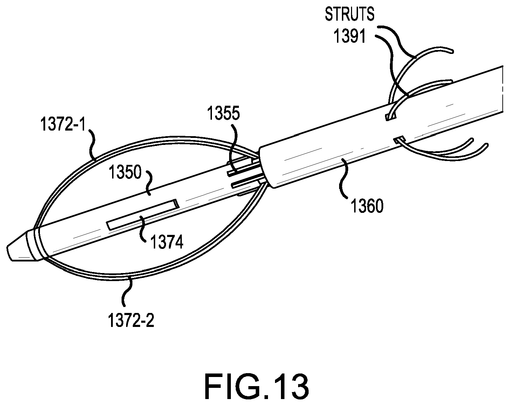

FIG. 13 illustrates centering wires in rotational vibrating debulking, in accordance with a representative embodiment.

In embodiments based on FIG. 11 and FIG. 12 vibrations are movements along the axis of the catheter. The vibrations along the axis of the catheter may be provided by two motors or by one motor with divided output. A first motor may continue to rotate the plurality of blades and a second motor may provide translational movement along the catheter. The second motor may be connected to the drive shaft assembly of the rotatable inner shaft 1150 or the rotatable inner shaft 1250 as well as to the accompanying fixed outer shaft. The second motor may use a slot system to turn rotational movement into quick back and forth movements along the catheter as seen in each of Fig. 11 and FIG. 12. The movement from two motors may alternatively be produced by a linear resonance actuator.

FIG. 13 shows a rotatable inner shaft 1350 projecting from a fixed outer shaft 1360. Rails 1355 are provided on the rotatable inner shaft 1350 so that trolleys which are unlabeled can be moved along the rails 1355 to move the plurality of blades 1372. Trolleys can be used to adjust a first rotatable blade 1372-1 and a second rotatable blade 1372-2 to project from the rotatable inner shaft 1350. A vibrational element 1374 is disposed on the rotatable inner shaft 1350. In FIG. 13, struts 1391 project from the fixed outer shaft 1360 on a proximal end and are used to assist in centering a catheter system in FIG. 13 in a vessel. The struts 1391 are a centering tool configured to center the catheter system in FIG. 13. The struts 1391 comprise a plurality of extensions from the rotatable inner shaft 1350 of the catheter and configured to expand and collapse by mechanism of the plurality of blades 1372. or a balloon inflated to a designated pressure to maintain a predetermined diameter.

In FIG. 13, to improve concentric lumen formation, the catheter is provided with centering wires as a centering tool. The centering tool may be constructed from multiple wire type extensions from the catheter which will interact with the vessel wall to centralize the catheter. The wires will be expanded and collapsed by the same mechanism of the plurality of blades.

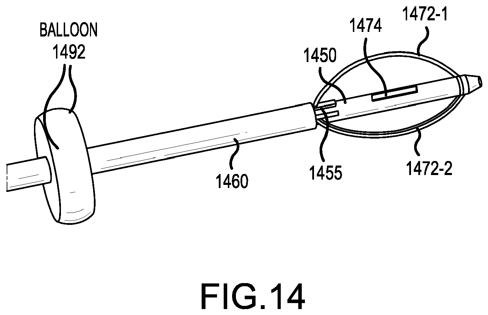

FIG. 14 illustrates balloon centering in rotational vibrating debulking, in accordance with a representative embodiment.

FIG. 14 shows a rotatable inner shaft 1450 projecting from a fixed outer shaft 1460. Rails 1455 are provided on the rotatable inner shaft 1450 so that trolleys which are unlabeled can be moved along the rails 1455 to move the plurality of blades 1472. Trolleys can be used to adjust a first rotatable blade 1472-1 and a second rotatable blade 1472-2 to project from the rotatable inner shaft 1450. A vibrational element 1474 is disposed on the rotatable inner shaft 1450. In FIG. 14, a balloon 1492 projects radially in every perpendicular radial direction from the fixed outer shaft 1460 to assist in centering a catheter system in FIG. 14 in a vessel. The balloon 1492 is a centering tool configured to center the catheter system in FIG. 14.

In FIG. 14, the catheter system is centralized by a balloon 1492. The balloon 1492 may be inflated to a designated pressure which will maintain a certain diameter. The diameter of the vessel may be controlled to expand and collapse by mechanism of the balloon inflated to a designated pressure to maintain a predetermined diameter.

The inflation tube will travel along the catheter system and the inflation lumen will be placed on the handle. The result is a balloon-centered rotational vibrating debulking device.

Accordingly, rotational vibrating debulking enables increased separation of disease from a vessel wall. A rotational vibrating debulking device may be provided with multiple blades which vibrate while rotating. The rotational vibrating debulking device will rotate via a motor in the proximal end of a catheter. The motor translates rotational movement through the catheter via a drive shaft. The vibrations may be produced by the rotating drive shaft by adding an eccentric rotating mass.

Although rotational vibrating debulking has been described with reference to several exemplary embodiments, it is understood that the words that have been used are words of description and illustration, rather than words of limitation. Changes may be made within the purview of the appended claims, as presently stated and as amended, without departing from the scope and spirit of rotational vibrating debulking in its aspects. Although rotational vibrating debulking has been described with reference to particular means, materials and embodiments, rotational vibrating debulking is not intended to be limited to the particulars disclosed; rather rotational vibrating debulking extends to all functionally equivalent structures, methods, and uses such as are within the scope of the appended claims. As one notable alternative embodiment, vibrations may be caused by an ultrasonic transducer rather than a vibrational element on a rotatable inner shaft. The ultrasonic transducer may turn changes in electrical signals to a mechanical vibration due to the properties of a piezoelectric material. When an electrical signal is generated over a piezoelectric material, the electrical signal will deform the material and the device will rapidly change voltage over the piezoelectric material and transfer vibrations to the plurality of blades. The results will include increased friction and improved cutting by the plurality of blades.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

### Additional embodiments

Embodiment 1. A catheter system, comprising:
a catheter having a distal end, a proximal end, and a longitudinal axis and comprising:
an inner shaft (530) having a vibrational element (574) disposed thereon;
an outer shaft (560) disposed around at least a proximal portion of the inner shaft;
a middle shaft (550) disposed between the inner shaft and the outer shaft; and
a plurality of blades (572), each having a proximal blade end and a distal blade end, the plurality of blades being disposed on the middle shaft (550) and configured to radially expand and compress with respect to the longitudinal axis;
wherein the inner shaft (530) and the middle shaft (550) are configured to rotate about the longitudinal axis; and
the outer shaft (560) is configured to translate with respect to the longitudinal axis in a longitudinal direction.

Embodiment 2. The catheter system of embodiment 1, further comprising:
a handle disposed at the proximal end of the catheter, the handle having an actuator configured to push each proximal blade end such that the plurality of blades radially expand with respect to the longitudinal axis.

Embodiment 3. The catheter system of embodiment 2, further comprising:
a first motor disposed in the handle and operatively connected to the inner shaft, and configured to rotate the inner shaft to generate vibrations in the blades.

Embodiment 4. The catheter system of embodiment 3, further comprising:
a second motor disposed in the handle and operatively connected to the middle shaft and configured to rotate the middle shaft about the longitudinal axis.

Embodiment 5. The catheter system of embodiment 2, wherein:
the inner shaft and the middle shaft are operatively connected to a single motor disposed in the handle, and the single motor is configured to rotate both the inner shaft at a first rotation speed to generate vibrations in the blades and to rotate the middle shaft at a second rotation speed to thereby rotate the plurality of blades.

Embodiment 6. The catheter system of embodiment 1, wherein the vibrational element comprises a piezoelectric transducer, a shape memory alloy, or an electroactive polymer.

Embodiment 7. The catheter system of embodiment 2, further comprising:
an actuator (motor, 560, 140) configured to push each proximal blade end such that the plurality of blades radially expand with respect to the longitudinal axis; and
a motor disposed in the handle and configured to rotate the inner shaft.

Embodiment 8. The catheter system of embodiment 1, wherein the vibrational element comprises an offset weight by which a center of mass of the catheter system is shifted radially with respect the longitudinal axis.

Embodiment 9. The catheter system of embodiment 1, wherein an amount of expansion from the longitudinal axis can be varied to change a diameter of the plurality of blades.

Embodiment 10. The catheter system of embodiment 1, wherein the outer shaft is configured to contact at least one of the plurality of blades at the distal end of the catheter.

Embodiment 11. The catheter system of embodiment 1, wherein the outer shaft comprises a circumferential groove configured to engage protrusions on top of the proximal end of each of the plurality of blades, wherein diameter of the plurality of blades is controlled by movement of the outer shaft relative to the middle shaft,
wherein the outer shaft is configured to control the plurality of blades to rotate freely.

Embodiment 12. The catheter system of embodiment 6, further comprising:
a centering tool (1391, 1492) configured to center the catheter, and comprising a plurality of extensions from the catheter and configured to expand and collapse or a balloon inflated to a designated pressure to maintain a predetermined diameter.

Embodiment 13. A method of operating a catheter system, comprising:
moving a catheter to a treatment zone, the catheter having a distal end, a proximal end, and a longitudinal axis, and comprising an inner shaft having a vibrational element disposed thereon, an outer shaft disposed around at least a proximal portion of the inner shaft, and a middle shaft disposed between the inner shaft and the outer shaft;
expanding a plurality of blades, each having a proximal end and a distal end, the plurality of blades being disposed on the middle shaft and configured to radially expand and compress with respect to the longitudinal axis; and
initiating vibration of the plurality of blades by rotating the inner shaft.

Embodiment 14. The method of embodiment 13, further comprising:
starting an actuator disposed in a handle disposed at the proximal end of the catheter and configured to translate the outer shaft to push each proximal end of the plurality of blades in a longitudinal direction such that the plurality of blades radially expand with respect to the longitudinal axis.

Embodiment 15. The method of embodiment 13, further comprising:
starting a first motor disposed in a handle at the proximal end of the catheter, the first motor being operatively connected to the inner shaft, and configured to rotate the inner shaft to generate vibrations in the blades.

## Claims

1. A catheter system, comprising:
a catheter having a distal end, a proximal end, and a longitudinal axis and comprising:
an inner shaft (530) having a vibrational element (574) disposed thereon;
an outer shaft (560) disposed around at least a proximal portion of the inner shaft;
a middle shaft (550) disposed between the inner shaft and the outer shaft; and
a plurality of blades (572), each having a proximal blade end and a distal blade end, the plurality of blades being disposed on the middle shaft (550) and configured to radially expand and compress with respect to the longitudinal axis;
wherein the inner shaft (530) and the middle shaft (550) are configured to rotate about the longitudinal axis; and
the outer shaft (560) is configured to translate with respect to the longitudinal axis in a longitudinal direction.

2. The catheter system of claim 1, further comprising:
a handle disposed at the proximal end of the catheter, the handle having an actuator configured to push each proximal blade end such that the plurality of blades radially expand with respect to the longitudinal axis.

3. The catheter system of claim 2, further comprising:
a first motor disposed in the handle and operatively connected to the inner shaft, and configured to rotate the inner shaft to generate vibrations in the blades.

4. The catheter system of claim 3, further comprising:
a second motor disposed in the handle and operatively connected to the middle shaft and configured to rotate the middle shaft about the longitudinal axis.

5. The catheter system of claim 2, wherein:
the inner shaft and the middle shaft are operatively connected to a single motor disposed in the handle, and the single motor is configured to rotate both the inner shaft at a first rotation speed to generate vibrations in the blades and to rotate the middle shaft at a second rotation speed to thereby rotate the plurality of blades.

6. The catheter system of claim 1, wherein the vibrational element comprises a piezoelectric transducer, a shape memory alloy, or an electroactive polymer.

7. The catheter system of claim 2, further comprising:
an actuator (motor, 560, 140) configured to push each proximal blade end such that the plurality of blades radially expand with respect to the longitudinal axis; and
a motor disposed in the handle and configured to rotate the inner shaft.

8. The catheter system of any one of claims 1 to 7, wherein the vibrational element comprises an offset weight by which a center of mass of the catheter system is shifted radially with respect the longitudinal axis.

9. The catheter system of any one of claims 1 to 8, wherein an amount of expansion from the longitudinal axis can be varied to change a diameter of the plurality of blades.

10. The catheter system of any one of claims 1 to 9, wherein the outer shaft is configured to contact at least one of the plurality of blades at the distal end of the catheter.

11. The catheter system of any one of claims 1 to 10, wherein the outer shaft comprises a circumferential groove configured to engage protrusions on top of the proximal end of each of the plurality of blades, wherein diameter of the plurality of blades is controlled by movement of the outer shaft relative to the middle shaft,
wherein the outer shaft is configured to control the plurality of blades to rotate freely.

12. The catheter system of any one of claims 1 to 11, further comprising:
a centering tool (1391, 1492) configured to center the catheter, and comprising a plurality of extensions from the catheter and configured to expand and collapse or a balloon inflated to a designated pressure to maintain a predetermined diameter.

13. A method of operating a catheter system, comprising:
moving a catheter to a treatment zone, the catheter having a distal end, a proximal end, and a longitudinal axis, and comprising an inner shaft having a vibrational element disposed thereon, an outer shaft disposed around at least a proximal portion of the inner shaft, and a middle shaft disposed between the inner shaft and the outer shaft;
expanding a plurality of blades, each having a proximal end and a distal end, the plurality of blades being disposed on the middle shaft and configured to radially expand and compress with respect to the longitudinal axis; and
initiating vibration of the plurality of blades by rotating the inner shaft.

14. The method of claim 13, further comprising:
starting an actuator disposed in a handle disposed at the proximal end of the catheter and configured to translate the outer shaft to push each proximal end of the plurality of blades in a longitudinal direction such that the plurality of blades radially expand with respect to the longitudinal axis.

15. The method of claim 13, further comprising:
starting a first motor disposed in a handle at the proximal end of the catheter, the first motor being operatively connected to the inner shaft, and configured to rotate the inner shaft to generate vibrations in the blades.
